# EUROPÄISCHE PATENTSCHRIFT

(11) **EP 2 514 396 B1**
(45) Veröffentlichungstag und Bekanntmachung des Hinweises auf die Patenterteilung: **20.08.2014**
(21) Anmeldenummer: 12002838.6
(22) Anmeldetag: 23.04.2012
(51) Int. Cl.: A61F 13/00, A61M 16/04

(54) **Zweilagige Kompresse, insbesondere Trachealkompresse**
Two-layer compress, in particular tracheal compress
Compresse double épaisseur, en particulier compresse trachéale

(30) Priorität: 21.04.2011 DE 202011005579 U
(43) Veröffentlichungstag der Anmeldung: 24.10.2012
(73) Patentinhaber: medi1one medical gmbh, 71336 Waiblingen (DE)
(72) Erfinder: Frick, Ulrich, 71404 KORB (DE)
(74) Vertreter: Kesselhut, Wolf

(56) Entgegenhaltungen:
- WO-A1-01/60422
- DE-C1- 3 430 994
- DE-C1- 19 728 328
- DE-U1- 29 710 855
- DE-U1-202005 015 474
- DE-U1-202005 015 555
- US-A- 3 416 525
- US-A- 5 013 307

## Beschreibung

Die Erfindung betrifft eine zweilagige Trachealkompresse, gemäß dem Oberbegriff von Anspruch 1.

Bei den heutzutage in der Medizin eingesetzten Tracheostomiekanülen und Endotrachealtuben, die beispielsweise zur künstlichen Beatmung von Patienten in deren Trachea eingesetzt werden, ist es von Vorteil, wenn die Zuleitungen in den Bereichen, in denen die Tuben in die künstlich erzeugten Körperöffnungen eintreten, von einer Kompresse umgebene sind, die die Tuben zum einen in ihrer Lage relativ zur Körperöffnung sichern, und zum anderen austretende Wundflüssigkeiten aufnehmen und ein Wundscheuem der Hautstellen um die Körperöffnungen herum verhindern.

In diesem Zusammenhang ist aus der DE 103 32 411 A1 eine mehrlagige Kompresse bekannt, die einen sich von der Durchtrittsöffnung zum Randbereich hin erstreckenden Schlitz aufweist, der über zueinander korrespondierende Eingriffsmittel formschlüssig gegen ein Auseinanderbewegen der Längskanten des Schlitzes gesichert werden kann, nachdem die Kompresse zuvor bei geöffnetem Schlitz um einen Tubus herum gelegt wurde. Obgleich die Schrift vorschlägt, die der Kleidung zugewandte Frontseite der Kompresse aus einem hydrophoben Material zu fertigen und die der Haut zugewandte Rückseite der Kompresse aus einem fussel- oder faserfreien Werkstoff herzustellen, gibt die Schrift keinen Hinweis darauf, wie die beiden Lagen miteinander verbunden sind.

Ähnliche zweilagige Trachealkompressen, die sich jedoch hinsichtlich der Ausgestaltung des Schlitzes unterscheiden, werden seit einiger Zeit von der Anmelderin der vorliegenden Anmeldung vertrieben, wobei die beiden Lagen der Kompressen mittels zweier Nähte, die rechts und links der Durchtrittsöffnung verlaufen, miteinander verbunden sind. Hierbei hat es sich als problematisch heraus gestellt, dass die Nähte oftmals zu einem Scheuem an den angrenzenden Hautpartien führen, was vom Patienten häufig als störend empfunden wird. Zudem ist die Herstellung der Kompressen, bei denen es sich um Massenprodukte handelt, vergleichsweise aufwändig und teuer, da stets zwei parallele Nähte in das Material eingebracht werden müssen, was den Herstellungsprozess verteuert.

Aus der DE 20 2005 015474 U1 ist eine Trachealkompresse bekannt, bei der eine erste körperseitige Lage aus Vliesstoff mit einer zweiten körperfernen Lage aus Vliesstoff über zwei randseitige Nähte miteinander verbunden sind. Durch das Vernähen der beiden Vlieslagen in den Randbereichen entstehen an den Nahtstellen Einschnürungen, die dazu führen können, dass die Randbereiche der Vlieslagen sich aufspreizen und die Randbereiche mit erhöhtem Druck auf den betreffenden Hautstellen eines Patienten aufliegen und auf diesen scheuern. Zudem ist das Vernähen der beiden Vlieslagen in fertigungstechnischer Hinsicht aufwendig.

Demgemäß ist es eine Aufgabe der vorliegenden Erfindung, eine Kompresse zu schaffen, die sich bei einem erhöhten Tragekomfort einfacher und kostengünstiger als Massenprodukt herstellen lässt.

Diese Aufgabe wird erfindungsgemäß durch eine Kompresse mit den Merkmalen von Anspruch 1 gelöst.

Weitere Merkmale der Erfindung sind in den Unteransprüchen beschrieben.

Die Erfindung umfasst eine geschlitzte und gelochte Trachealkompresse mit einer ersten Lage aus einem hydrophoben Werkstoff, z.B. einem Zellstoff. Die erste Lage, die bei angelegter Kompresse auf der dem Körper abgewandten Seite der Kompresse angeordnet ist, ist mit einer zweiten Lage aus einem Vliesstoff, z.B. einem Polyestervlies, durch Klebstoff, beispielsweise einen wasserunlöslichen und dauerklebrigen Klebstoff verbunden.

Soweit nachfolgend von einer ersten Lage aus einem hydrophobem Werkstoff gesprochen wird, so kann dies auch eine erste Lage bestehend aus einem Verbund aus einer oder mehreren Einzellagen aus saugfähigem Vliesstoff mit einschließen, die durch ein Ineinandergreifen der Vliesfäden untereinander verbunden sind, und die ausschließlich auf ihrer dem Körper abgewandten Außenseite mit einer flüssigkeitsundurchlässigen Beschichtung oder einer Funktionsmembran versehen werden, welche lediglich für Wasserdampf, nicht jedoch für Flüssigkeiten durchlässig ist,

In entsprechender Weise kann die zweite Lage aus Vliesstoff ebenfalls auch einen Verbund aus Vliesstoff umfassen, bei dem mehrere Einzellagen aus einem saugfähigen Vliesstoff durch ein Ineinandergreifen der Vliesfäden untereinander verbunden sind.

Durch die Erfindung ergibt sich der Vorteil, dass die Trachealkompresse auf ihrer dem Körper zugewandten Seite keine Bereiche erhöhter Steifigkeit mehr aufweist, die zu einer verstärkten Reizung der angrenzenden Hautpartien führen, wie dies bei den eingangs erwähnten Kompressen der Fall ist, bei denen die erste und die zweite Lage durch Vernähen entlang zweier Nahtstellen miteinander verbunden sind.

Als Klebstoff kommt vorzugsweise ein hochviskoser und dauerklebriger Haftklebstoff zum Einsatz, wie er insbesondere von doppelseitigen Klebebändern her bekannt ist, obgleich auch andere Arten von Klebstoffen einsetzbar sind. Durch den Einsatz von dauerelastischen Haftklebstoffen, d.h. Klebstoffen ohne Verfestigungsmechanismus, die nicht vollständig aushärten, sondern ihre Klebrigkeit auch über einen längeren Zeitraum hinweg beibehalten, ergibt sich der Vorteil, dass sich die Elastizität - und dadurch der Tragekomfort - der Kompressen im Bereich der Klebestellen nach dem Verbinden der beiden Lagen nicht verringert. Die Klebstoffe sind bevorzugt feuchtigkeitsbeständige medizinische Klebstoffe, wie sie z.B. bei Heftpflastern eingesetzt werden, die insbesondere auch hautverträglich und nicht toxisch sind.

Bei einem Ausführungsbeispiel der Erfindung ist der Klebstoff als Klebstofflage in Form von zwei im Abstand zueinander angeordneten Klebstoffstreifen auf die erste und zweite Lage aufgebracht, wobei die Klebstoffstreifen im Bereich zweier einander gegenüberliegender Randabschnitte der Trachealkompresse positioniert sind. Durch das Aufbringen des Klebstoffes entlang zweier Klebstoffstreifen, die sich bezogen auf die angelegte Kompresse bevorzugt jeweils entlang eines der beiden vertikalen Längsränder der Kompresse erstrecken, jedoch ebenso in Querrichtung verlaufen können, ergibt sich der Vorteil, dass der Tragekomfort durch die gegenüber Nähten breiteren, mit Klebstoff versehenen Verbindungsbereiche auch dadurch verbessert wird, dass die Randbereiche nicht mehr auseinander klaffen, wie dies oftmals bei den zuvor erwähnten genähten Kompressen zu beobachten ist, bei denen die Nähe das elastische Material der beiden Lagen entlang der Nähte einschnüren und die Randbereiche dadurch voneinander abheben.

Die Klebstoffstreifen können jeweils beispielsweise eine Breite von 0,5 cm bis 2 cm besitzen, wodurch sich ein guter Kompromiss zwischen einer zuverlässigen Verbindung der beiden Lagen und einem hohen Tragekomfort ergibt. Der Tragekomfort wird hierbei gegenüber einer vollflächigen Verklebung dadurch zusätzlich verbessert, dass der Kraftschluss zwischen den beiden Lagen lediglich entlang der streifenförmigen Bereiche, und nicht über die gesamte Fläche hinweg, besteht, was zu einer Verringerung der Eigensteifigkeit der Kompresse insgesamt führt.

Nach einem weiteren der Erfindung zu Grunde liegenden Gedanken verlaufenden die Klebstoffstreifen bevorzugt in einem Abstand von den seitlichen Rändern der ersten und zweiten Lage, der - vom Seitenrand des Klebestreifens aus gerechnet - bevorzugt im Bereich zwischen 1 mm und 10 mm, insbesondere im Bereich von 5 bis 7 mm, liegt. Hierdurch ergibt sich der Vorteil, dass die Kompresse im Randbereich nicht zu einem Scheuern neigt, da die ursprünglichen elastischen Eigenschaften des Materials der dem Körper zugewandten zweiten Lage aus Vlies erhalten bleiben und nicht durch die Verbindung mit der ersten Lage nachteilig beeinträchtigt werden.

Bei der erfindungsgemäßen Trachealkompresse umfasst die Klebstofflage ein filmartiges Trägermaterial, welches beiderseits mit Klebstoff beschichtet ist. Das beidseitig mit Klebstoff beschichtete Trägermaterial ist hierbei bevorzugt als doppelseitiges Klebeband ausgeführt, das z.B. von einer Rolle auf die Innenseite einer der beiden Lagen der Kompresse abgerollt wird. Das Aufbringen des doppelseitigen Klebebandes kann hierbei in vorteilhafter Weise fortlaufend maschinell erfolgen, wodurch sich der Herstellungsprozess der erfindungsgemäßen Kompressen in vorteilhafter Weise beschleunigt und die Herstellungskosten reduziert werden. Die andere Lage der Kompresse, bevorzugt die Lage aus Vliesstoff, wird nach dem Entfernen der Schutzlage, die die zweite, mit Klebstoff beschichtete Seite des Trägermaterials abdeckt, bevorzugt ebenfalls fortlaufend über die erste Lage der Kompresse geführt und beispielsweise durch eine geeignete Walze angedrückt, so dass eine zuverlässige Klebeverbindung entlang des streifenförmigen Klebebereichs erhalten wird. Wie bereits zuvor ausgeführt wurde, werden bevorzugt zwei streifenförmige Klebebereiche aus doppelseitigem Klebeband in geringem Abstand von den seitlichen Längsränder der Kompresse angeordnet, was zu dem weiteren Vorteil führt, dass bei der maschinellen Herstellung zwischen den Abrollvorrichtungen für die doppelseitigen Klebebänder, bzw. den Auftragsvorrichtungen für den Klebstoff ausreichend Platz vorhanden ist.

Alternativ zu dem zuvor beschriebenen Auftrag des Klebstoffs entlang zweier streifenförmiger Abschnitte kann es ebenfalls vorgesehen sein, dass die Klebstofflage eine Vielzahl von flächigen Klebeabschnitten umfasst, die bevorzugt im Wesentlichen gleichmäßig verteilt zwischen der ersten und zweiten Lage angeordnet sind. Diese Klebeabschnitte können durch Aufbringen von Abschnitten aus Klebeband oder durch ein lokales Aufbringen, z.B. Aufsprühen von Klebstoffpunkten mit einem Durchmesser von beispielsweise 1 cm erzeugt werden. Durch das Vorsehen von einer Vielzahl von gleichmäßig über die erste und zweite Lage hinweg verteilten Klebeabschnitten ergibt sich der Vorteil, dass bei einer sehr guten Verbindung der Lagen die Elastizität der Kompresse im Wesentlichen homogen über die erste und zweite Lage hinweg erhalten bleibt, da durch die zwischen den Klebeabschnitten liegenden, nicht durch Klebstoff verbundenen Bereiche, ein Gleiten des aneinander angrenzenden Vlieslagen ermöglicht wird, was eine lokale Verschiebung der aneinander anliegenden Oberflächen der ersten und zweiten Lage erlaubt.

Nach einem weiteren bevorzugten der Erfindung zugrunde liegenden Gedanken kann die Elastizität der Trachealkompresse - und damit deren Tragekomfort - dadurch noch weiter erhöht werden, dass die Klebstofflage, insbesondere das Trägermaterial der Trägerschicht des doppelseitigen Klebebandes, mit einer Vielzahl von Perforationen versehen ist, die über die Trägerschicht hinweg in vorteilhafter Weise im Wesentlichen homogen verteilt sind. Die Perforationen werden bevorzugt bereits bei der Herstellung des Klebebandes in Form von Löchern in die Trägerschicht eingebracht und können beispielsweise einen Durchmesser im Bereich von 1 mm bis 4 mm besitzen.

Bei einer bevorzugten Ausführungsform der Erfindung besitzt die Kompresse eine zentrale Durchfuhröffnung für einen Trachealtubus und vorzugsweise auch einen sich von der Durchführöffnung aus zu einem der seitlichen Ränder der ersten und zweiten Lage erstreckenden Schlitz, entlang von welchem die erste und zweite Lage durchbrochen sind, um den Trachealtubus beim Anlegen der Kompresse von der Seite her in die Durchfuhröffnung einfädeln zu können. Bei dieser Ausführungsform der Erfindung ist der Klebstoff zum Verbinden der beiden Lagen zumindest abschnittsweise auch im Bereich des Schlitzes auf die erste und/oder zweite Lage aufgebracht, wobei der Bereich, in dem der Klebstoff aufgebracht ist, sich bevorzugt in Querrichtung über den Schlitz hinweg erstreckt. Hierdurch ergibt sich der Vorteil, dass die erste und die zweite Lage im Bereich des Schlitzes nach dem Einfädeln des Trachealtubus durch den Schlitz hindurch in Querrichtung zum Schlitz wieder miteinander über die Klebestelle verbunden werden können. Hierbei kommt bevorzugt ein dauerelastischer Klebstoff zum Einsatz, wie er beispielsweise auf dem Trägermaterial von Klebebändern verwendet wird.

Bei der zuvor beschriebenen bevorzugten Ausführungsform der Erfindung ist es weiterhin von Vorteil, wenn der Schlitz und/oder die Durchtrittsöffnung nach dem Verkleben der ersten und zweiten Lage der Kompresse miteinander als Stanznut in die erste und zweite Lage eingebracht werden und die Stanznut durch wenigstens einen mit Klebstoff beschichteten Abschnitt verläuft, der im Bereich des Schlitzes angeordnet ist. Hierdurch lassen sich mit vergleichsweise geringem Herstellungsaufwand geschlitzte und mit einer Durchtrittsöffnung versehene Trachealkompressen fertigen, die sich beim Einsatz von dauerklebrigem Klebstoff nach dem Einfädeln des Trachealtubus wieder zuverlässig durch Zusammenführen der aneinander haftenden Klebstoffabschnitte auf einander gegenüberliegenden Seiten des Schlitzes verschließen lassen. Die zuvor beschriebene bevorzugte Ausführungsform besitzt den weiteren Vorteil, dass die Klebestellen auf den einander gegenüberliegenden Seiten des Schlitzes unmittelbar nach dem Einbringen der Stanznut und dem Entfernen des Stanzmessers aufgrund der freigesetzten frischen Klebstoffstellen im Bereich des Schlitzes vergleichsweise gut aneinander haften. Dies führt dazu, dass der Schlitz unmittelbar nach der Herstellung der Kompresse durch eine vergleichsweise gut haftende Klebeverbindung der einander gegenüberliegenden Klebestellen verschlossen ist und dadurch die Kompresse die Funktion einer ungeschlitzten Kompresse mit Durchtrittsöffnung besitzt.

Alternativ zu dem Einsatz als ungeschlitzte Trachealkompresse ist es jedoch ebenfalls möglich, den Schlitz im Bereich der Klebestellen unmittelbar vor dem Einfädeln die Kompresse durch ein kräftigeres Auseinanderziehen der Klebestellen zu öffnen und die Kompresse anschließend im Wesentlichen kräftefrei über den geöffneten Schlitz auf einen gelegten Trachealtubus aufzufädeln. Da die beiden Klebestellen beim Zusammenführen des Vliesmaterials der ersten und zweiten Lage nach dem Einfädeln zwangsweise auch mit dem Vliesstoff in Berührung gelangen, wird die Klebewirkung zwischen den beiden Klebestellen durch die anhaftenden Vliesfasern stark herab gesetzt, so dass die beiden Klebestellen zwar nach dem Schließen des Schlitzes weiterhin verbunden bleiben und die Kompresse gegen ein Herunterfallen sichern, jedoch bei Bedarf mit einer erheblich geringeren Kraft wieder geöffnet werden können, um die erfindungsgemäße Kompresse nach ihrem Gebrauch wieder zu entfernen.

Zusätzlich oder alternativ zu der durch den Schlitz getrennten Klebestelle kann bei der zuvor beschriebenen Ausführungsform der erfindungsgemäßen Kompresse auch ein mechanischer Verschluss vorgesehen sein, der beispielsweise durch einen zick-zackförmigen Verlauf des Schlitzes bewirkt werden kann.

Die Erfindung wird nachfolgend mit Bezug auf die Zeichnungen anhand einer bevorzugten Ausführungsform beschrieben.

In den Zeichnungen zeigen:
- Fig. 1: eine schematische Aufsicht auf eine bevorzugte Ausführungsform einer erfindungsgemäßen Trachealkompresse und
- Fig. 2: eine Querschnittsansicht der Trachealkompresse von Fig. 1.

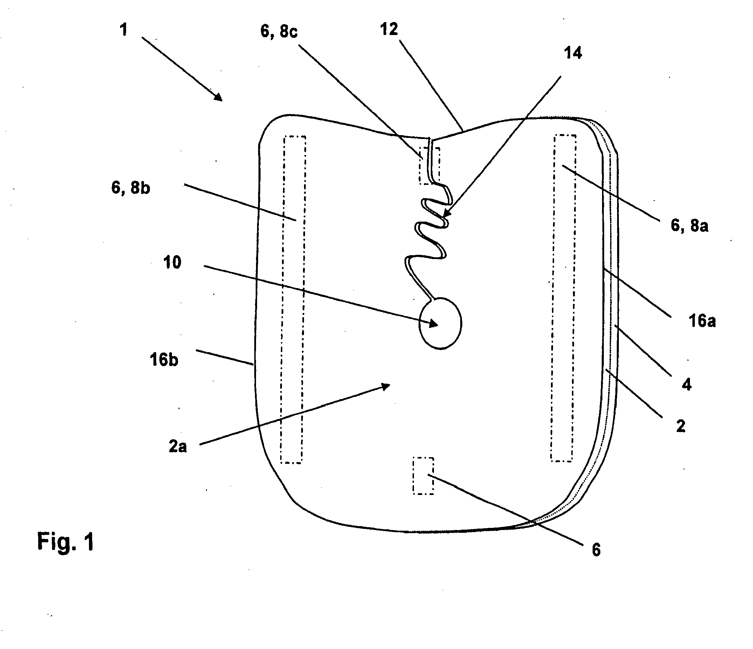

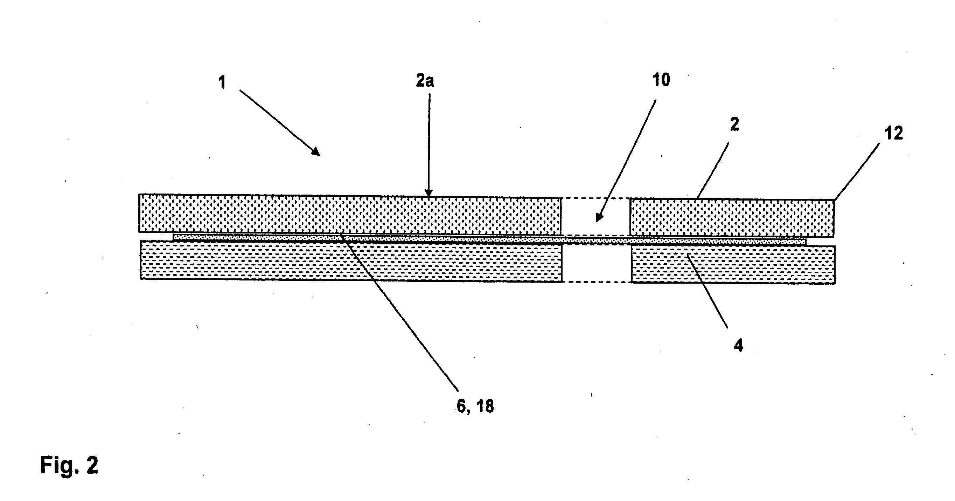

Wie in den Figuren 1 und 2 gezeigt ist, umfasst die Trachealkompresse 1 eine erste Lage 2, die aus einem vorzugsweise hydrophoben Werkstoff besteht, der z.B. ein auf seiner dem Körper abgewandten Außenseite 2a mit einer flüssigkeitsundurchlässigen Funktionsschicht versehener Vliesstoff ist. Die Kompresse 1 umfasst weiterhin eine zweite Lage 4 aus einem Flüssigkeiten absorbierenden Vliesstoff, der z.B. aus verpressten Baumwoll- oder Viskosefasern besteht. Die erste Lage 2 und die zweite Lage 4 besitzen bevorzugt im Wesentlichen die gleiche Dicke und sind durch Klebstoff miteinander verbunden, der als Klebstofflage 6 in Form von zwei im Abstand zueinander angeordneten Klebstoffstreifen 8a und 8b zwischen den aneinander angrenzenden Innenflächen der ersten und zweiten Lage 2, 4 vorgesehen ist.

Wie der Darstellung von Fig. 1 weiterhin im Detail entnommen werden kann, besitzt die Kompresse im Bereich ihres Zentrums eine Durchtrittöffnung 10 für einen nicht näher gezeigten Trachealtubus, der über einen sich von der Durchtrittsöffnung 10 zu einem oberen Rand 12 erstreckenden Schlitz 14 in die Durchtrittsöffnung 10 eingefädelt werden kann. Der Schlitz 14 besitzt bevorzugt eine dargestellte wellenlinienartige Form, die dafür sorgt, dass die beiden Lagen 2, 4 im Bereich des Schlitzes 14 formschlüssig ineinander greifen, um die Kompresse während ihres Gebrauchs gegen ein Herabfallen am Tubus zu sichern.

Gemäß der Darstellung von Fig. 1 erstrecken sich die Klebstoffstreifen 8a, 8b entlang und im Wesentlichen parallel zu den beiden vertikalen Längsrändern 16a, 16b der Kompresse 1, wobei der in Fig. 1 übertrieben dargestellte Abstand zwischen dem jeweiligen Längsrand 16a, 16b und dem Rand des zugehörigen Klebstoffstreifens 8a, 8b im Bereich von z.B. 1 mm liegt.

Wie der Fig. 1 weiterhin zu entnehmen ist, kann im Bereich des Schlitzes 14 ein Klebstoffstreifen 8c angeordnet sein, der in gleicher Weise wie die beiden seitlichen Klebstoffstreifen 8a und 8b aus einem doppelseitigen Klebeband besteht, welches eine Trägerschicht 18 umfasst, die - wie in Fig. 2 angedeutet - beidseitig mit einem dauerelastischen und dauerklebrigen Klebstoff (6) beschichtet ist. Derartige doppelseitige Klebebänder sind für medizinische Anwendungen bekannt und weisen ein Trägermaterial auf, welches - in gleicher Weise wie z.B. bei Heftpflastern - mit einem dauerelastischen, dauerklebrigen, wasserunlöslichen und nicht toxischen hautfreundlichen Klebstoff beschichtet ist.

### Liste der Bezugszeichen

- 1: Trachealkompresse
- 2: erste Lage
- 2a: Außenseite der ersten Lage
- 4: zweite Lage
- 6: Klebstoff/Klebstofflage
- 8a: Klebstoffstreifen
- 8b: Klebstoffstreifen
- 8c: Klebstoffstreifen im Bereich des Schlitzes
- 10: Durchtrittsöffnung
- 12: oberer Rand
- 14: Schlitz
- 16a: Längsrand
- 16a: Längsrand
- 18: Trägerschicht

## Patentansprüche

1. Trachealkompresse mit einer ersten Lage (2) aus einem saugfähigen Vliesstoff, die ausschließlich auf ihrer dem Körper abgewandten Außenseite (2a) mit einer flüssigkeitsundurchlässigen Beschichtung versehen ist, und einer mit der ersten Lage (2) verbundenen zweiten Lage (4) aus einem saugfähigen Vliesstoff, wobei die erste Lage (2) und die zweite Lage (4) durch eine Klebstofflage (6) miteinander verbunden sind,
**dadurch gekennzeichnet,**
**dass** die Klebstofflage (6) ein filmartiges Trägermaterial (18) umfasst, welches beiderseits mit einem dauerklebrigen Klebstoff beschichtet ist, und
**dass** die Klebstofflage (6) in Form von zwei im Abstand zueinander angeordneten Klebstoffstreifen (8a, 8b) vorgesehen ist, die beabstandet voneinander im Bereich zweier gegenüberliegender Längsränder (16a, 16b) der Kompresse (1) verlaufen.

2. Kompresse nach Anspruch 1,
**dadurch-gekennzeichne**
dass sich die Klebstoffstreifen (8a, 8b), bezogen auf die angelegte Kompresse (1), entlang der vertikalen Längsränder (16a, 16b) erstrecken.

3. Kompresse nach Anspruch 1 oder 2,
**dadurch gekennzeichnet,**
**dass** die Klebstoffstreifen (8a, 8b) in einem Abstand von den seitlichen Längsrändern (16a, 16b) der ersten und zweiten Lage (2, 4) verlaufen.

4. Kompresse nach Anspruch 3,
**dadurch gekennzeichnet,**
**dass** der Abstand zwischen dem Längsrand (16a, 16b) der erste und zweiten Lage (2, 4) und dem angrenzenden Längsrand des Klebstoffstreifens (8a, 8b) im Bereich zwischen 1 mm und 10 mm, insbesondere im Bereich von 5 bis 7 mm, liegt.

5. Kompresse nach Anspruch 1,
**dadurch gekennzeichnet,**
**dass** das beidseitig mit Klebstoff (6) beschichtete Trägermaterial (18) ein doppelseitiges Klebeband ist, dass auf die Innenseite einer Lage (2, 4) aufgebracht ist, und dass die andere Lage (4, 2) der Kompresse (1) nach dem Entfernen einer nichthaftenden Schutzlage vom Klebeband auf die zweite Seite des Trägermaterials (18) aufgeklebt ist.

6. Kompresse nach einem der vorhergehenden Ansprüche,
**dadurch gekennzeichnet,**
**dass** die Klebstofflage (6) Perforationen aufweist.

7. Kompresse nach einem der vorhergehenden Ansprüche,
**dadurch gekennzeichnet,**
**dass** diese eine Durchführöffnung (10) aufweist.

8. Kompresse nach Anspruch 7,
**dadurch gekennzeichnet,**
**dass** diese eine Durchführöffnung (10) sowie einen sich von der Durchführöffnung aus zu einem der Ränder (12) der ersten und zweiten Lage (2, 4) erstreckenden Schlitz (14) aufweist, entlang von welchem die erste und zweite Lage (2, 4) durchbrochen ist, und dass der Klebstoff (6) ein dauerklebriger Klebstoff ist, der zum Verbinden der beiden Lagen (2, 4) zumindest teilweise auch im Bereich des Schlitzes (14) auf die erste und/oder zweite Lage (2, 4) aufgebracht ist.

## Claims

1. Tracheal compress with a first layer (2) comprising an absorbent nonwoven material which is provided only on its outer side (2a) facing away from the body with a liquid-impermeable coating and a second layer (4) connected to the first layer (2) and comprising an absorbent nonwoven material, wherein the first layer (2) and the second layer (4) are joined together by means of an adhesive layer (6),
**characterised in that**
the adhesive layer (6) comprises a film-like carrier material (18) which is coated on both sides with a permanently sticky adhesive, and that the adhesive layer (6) is provided in the form of two adhesive strips (8a, 8b) spaced apart from each other and which run, with a gap in between, in the area of two opposite longitudinal edges (16a, 16b) of the compress (1).

2. Compress according to claim 1,
**characterised in that,**
in relation to the applied compress (1), the adhesive strips (8a, 8b) extend along the vertical longitudinal edges (16a, 16b).

3. Compress according to claim 1 or 2,
**characterised in that**
the adhesive strips (8a, 8b) run with a gap from the lateral longitudinal edges (16a, 16b) of the first and second layers (2, 4).

4. Compress according to claim 3,
**characterised in that**
the distance between the longitudinal edge (16a, 16b) of the first and second layers (2, 4) and the adjacent longitudinal edge of the adhesive strips (8a, 8b) falls in the range between 1 mm and 10 mm, in particular in the range of 5 to 7 mm.

5. Compress according to claim 1,
**characterised in that**
the carrier material (18) coated on both sides with adhesive (6) is a double-sided adhesive tape which is applied on the inner side of a layer (2, 4), and that the other layer (4, 2) of the compress (1) is glued on to the second side of the carrier material (18) after removal of a non-stick protective layer from the adhesive tape.

6. Compress according to one of the preceding claims,
**characterised in that**
that the adhesive layer (6) has perforations.

7. Compress according to one of the preceding claims,
**characterised in that**
it has one insertion opening (10).

8. Compress according to claim 7,
**characterised in that**
it has an insertion opening (10) and a slit (14) extending out from the insertion opening to one of the edges (12) of the first and second layers (2, 4), along which the first and second layers (2, 4) are split and that the adhesive (6) is a permanently sticky adhesive which is applied to the first and / or second layer (2, 4) to join the two layers 2, 4 at least partially in the region of the slit (14) also.

## Revendications

1. Compresse trachéale avec une première couche (2) en tissu non tissé absorbant, qui est dotée d'un revêtement imperméable aux liquides exclusivement sur sa face extérieure (2a) opposée au corps, et avec une deuxième couche (4) en tissu non tissé absorbant reliée à la première couche (2), dans laquelle la première couche (2) et la deuxième couche (4) sont reliées ensemble par le biais d'une couche d'adhésif (6),
**caractérisée en ce**
**que** la couche d'adhésif (6) comprend un matériau support de type film (18), lequel est revêtu des deux côtés par un adhésif permanent, et en ce que la couche d'adhésif (6) est prévue sous la forme de deux bandes d'adhésif (8a, 8b) disposées à distance l'une de l'autre, qui sont espacées l'une de l'autre dans la zone de deux bordures longitudinales opposées (16a, 16b) de la compresse (1).

2. Compresse selon la revendication 1,
**caractérisée en ce**
**que** les bandes d'adhésif (8a, 8b), par rapport à la compresse posée (1), s'étendent le long des bordures longitudinales verticales (16a, 16b).

3. Compresse selon la revendication 1 ou 2,
**caractérisée en ce**
**que** les bandes d'adhésif (8a, 8b) sont à distance des bordures longitudinales latérales (16a, 16b) de la première et de la deuxième couche (2, 4).

4. Compresse selon la revendication 3,
**caractérisée en ce**
**que** la distance entre la bordure longitudinale (16a, 16b) de la première et de la deuxième couche (2, 4) et la bordure longitudinale adjacente de
la bande d'adhésif (8a, 8b) se situe dans la plage comprise entre 1 mm et 10 mm, en particulier dans la plage de 5 à 7 mm.

5. Compresse selon la revendication 1,
**caractérisée en ce**
**que** le matériau support (18) revêtu des deux côtés par l'adhésif (6) est un ruban adhésif double face, qui est appliqué sur la face intérieure d'une couche (2, 4), et en ce que l'autre couche (4, 2) de la compresse (1) est collée par le ruban adhésif sur la deuxième face du matériau support (18) après le retrait d'une couche de protection non adhérente.

6. Compresse selon l'une des revendications précédentes,
**caractérisée en ce**
**que** la couche d'adhésif (6) présente des perforations.

7. Compresse selon l'une des revendications précédentes,
**caractérisée en ce**
**qu'**elle présente une ouverture de passage (10).

8. Compresse selon la revendication 7,
**caractérisée en ce**
**qu'**elle présente une ouverture de passage (10) ainsi qu'une fente (14) s'étendant depuis l'ouverture de passage vers l'un des bords (12) de la première et de la deuxième couche (2, 4), le long de laquelle la première et la deuxième couche (2, 4) sont percées, et en ce que l'adhésif (6) est un adhésif permanent, qui est appliqué sur la première et/ou la deuxième couche (2, 4) pour relier les deux couches (2, 4) au moins partiellement également dans la zone de la fente (14).
